# EUROPEAN PATENT APPLICATION

(11) **EP 3 575 413 A1**
(43) Date of publication of application: **04.12.2019**
(21) Application number: 19170821.3
(22) Date of filing: 06.12.2010
(51) Int. Cl.: C12Q 1/68, C12Q 1/6886

(54) **PHOSPHATIDYLINOSITOL-3-KINASE PATHWAY BIOMARKERS**

(30) Priority: 11.12.2009 US 28582109 P; 18.12.2009 US 28787209 P
(62) Divisional of application: 10805335.6
(71) Applicant: Wyeth LLC, New York, NY 10017 (US)
(72) Inventor: BERKENBLIT, Anna, Needham, MA 02492 (US); COUGHLIN, Christina Marie, Berwyn, PA 19312 (US); FEINGOLD, Jay Marshall, Wynnewood, PA 19096 (US); JOHNSTON, Daniel Stephen, Trappe, PA 19426 (US); STRAHS, Andrew Louis, Maynard, MA 01754 (US); ZACHARCHUK, Charles, Westford, MA 01886 (US)
(74) Representative: Jones Day

(57) **Abstract**

The invention provides a composition comprising neratinib for use in a method for treatment of breast cancer in a subject, wherein the subject is positive for the presence of one or more of: PIK3ACA gene amplification, a mutation in PIK3CA and a decrease in PTEN protein expression; and wherein the method further comprises administration of capecitabine.

## Description

This application claims the benefit of United States Application No. 61/285,821, filed December 11, 2009, and United States Application No. 61/287,872, filed December 18, 2009, both of which are hereby incorporated by reference in their entirety.

### FIELD OF INVENTION

The present disclosure relates to methods for treating breast cancer. The cancer may be resistant to treatment with one or more known breast cancer treatment drugs. The present disclosure also provides a patient selection strategy (i.e., identify patients with "*P*/*3K activated*" tumors) for predicting patient response to drug therapy. The disclosure is also related to methods of treating breast cancer patients with a pan-ErbB tyrosine kinase inhibitor.

### BACKGROUND OF INVENTION

Constitutive PI3K activation in human cancer is thought to contribute to drug resistance to targeted agents and standard cytotoxic therapy. The combination of activation mechanisms and the multiple downstream cascades that emanate from the PI3K node contribute to the difficulty in measuring PI3K activation as a biomarker.

Neratinib is an orally available, 6,7-disubstituted-4-anilinoquinoline-3-carbonitrile irreversible inhibitor of the HER-2 receptor tyrosine kinase with potential antineoplastic activity. Neratinib binds to the HER-2 receptor irreversibly, thereby reducing autophosphorylation in cells, apparently by targeting a cysteine residue in the ATP-binding pocket of the receptor. Treatment of cells with this agent results in inhibition of downstream signal transduction events and cell cycle regulatory pathways; arrest at the G1-S (Gap 1/DNA synthesis)-phase transition of the cell division cycle; and ultimately decreased cellular proliferation. Neratinib also inhibits the epidermal growth factor receptor (EGFR) kinase and the proliferation of EGFR-dependent cells.

Trastuzumab (Herceptin) is a monoclonal antibody that interferes with the HER2/Neu HER2/neu receptor.The HER receptors are proteins that are embedded in the cell membrane and communicate molecular signals from outside the cell to inside the cell, and turn genes on and off. The HER proteins regulate cell growth, survival, adhesion, migration, and differentiation-functions that are amplified or weakened in cancer cells. In some cancers, notably some breast cancers, the HER2 receptor is defective and stuck in the "on" position, and causes breast cells to reproduce uncontrollably, causing breast cancer.

### SUMMARY OF INVENTION

In some embodiments, the invention provides methods for treating breast cancer in a subject which comprise obtaining a sample from the subject; detecting the presence or absence of one or more of PIK3CA gene amplification; a mutation in PIK3CA; and a decrease in PTEN protein expression; and treating a patient that is positive for the presence of one or more of PIK3CA gene amplification; a mutation in PIK3CA; and a decrease in PTEN protein expression by administering a pan-ErbB tyrosine kinase inhibitor.

In some embodiments, the pan-ErbB inhibitor is irreversible and prevents binding of PIK3CA to the intracellular portion of the ErbB receptor and in some embodiments the intracellular inhibitor of ErbB receptor tyrosine kinases is neratinib.

In some embodiments the invention provides methods of treatment as described herein where the mutation in the PIK3CA gene comprises one or more of the following point mutations: in exon 9 E is substituted with K at position 542 of the mature protein sequence; E with K or D at amino acid 545; and in exon 20 H is substituted with R at amino acid 1047 of the mature protein sequence.

In some embodiments, detection of the mutation in the PIK3CA gene comprises a Polymerase Chain Reaction (PCR) assay, or direct nucleic acid sequencing or hybridization with a nucleic acid probe specific for the PIK3CA gene. In some embodiments, the detection of PTEN expression comprises one or more of: reverse phase protein array, western blotting, semi-quantitative or quantitative IHC.

In some embodiments the invention provides methods for treating breast cancer in a subject which comprise obtaining a sample from the subject; detecting the presence or absence of one or more of PIK3CA gene amplification; a mutation in PIK3CA; and a decrease in PTEN protein expression; and treating a patient that is positive for the presence of one or more of PIK3CA gene amplification; a mutation in PIK3CA; and a decrease in PTEN protein expression by administering an pan-ErbB inhibitor and which further comprise administering one or more compositions or therapies to the subject if the subject is positive for PIK3CA gene amplification wherein the compositions or therapies are useful for treating breast cancer. The additional treatment can comprise one or more of surgery, radiation or additional chemotherapy agents selected from one or more of the following: aromatase inhibitors, including letrozole (Femara), anastrazole (Arimidex), fulvestrant (Faslodex) and exemestane (Aromasin); goserelin (Zoladex); anthracyclines, including doxorubicin (Adriamycin), epirubicin (Ellence), and liposomal doxorubicin (Doxil); taxanes, including docetaxel (Taxotere), paclitaxel (Taxol), and protein-bound paclitaxel (Abraxane), Cyclophosphamide (Cytoxan); Capecitabine (Xeloda) and 5 fluorouracil (5 FU); Vinorelbine (Navelbine); Gemcitabine (Gemzar);Trastuzumab (Herceptin), lapatinib, BIBW2992, PI3K inhibitors (e.g., XL147, PX-866), mTOR inhibitors (e.g., temsirolimus, everolimus), and dual PI3K-mTOR inhibitors (e.g., BEZ235). In some embodiments the invention provides methods for treating breast cancer in a subject which comprise obtaining a sample from the subject; detecting the presence or absence of one or more of PIK3CA gene amplification; a mutation in PIK3CA; and a decrease in PTEN protein expression; and treating a patient that is negative for all three of these biomarkers with Trastuzumab.

In some embodiments, the invention provides methods of treating a breast cancer subject which comprise detecting the presence or absence of one or more of PIK3CA gene amplification; a mutation in PIK3CA; and a decrease in PTEN protein expression; wherein if a subject is negative for PIK3CA gene amplification; a mutation in PIK3CA; and a decrease in PTEN protein expression the subject is administered Trastuzumab.

In some embodiments, the invention provides methods for determining if a subject with breast cancer is a candidate for treatment with a pan-ErbB tyrosine kinase inhibitor which comprises: obtaining a sample from the subject; detecting the presence or absence of PIK3CA gene amplification; wherein if the subject is positive for the presence of one or more of the following: PIK3CA gene amplification; a mutation in PIK3CA; and a decrease in PTEN protein expression, then the subject is a identified as a candidate for treatment with a pan-ErbB tyrosine kinase inhibitor. In some embodiments, the pan-ErbB inhibitor is irreversible and prevents binding of PIK3CA to the intracellular portion of the ErbB receptor and in some embodiments the intracellular inhibitor of ErbB receptor tyrosine kinases is neratinib.

In some embodiments, the methods for determining if a subject is a candidate for treatment with a pan-ErbB tyrosine kinase inhibitor or e.g., neratinib comprise detecting a mutation in the PIK3CA gene is selected from the following point mutations: in exon 9 E is substituted with K at position 542 of the protein sequence; in exon 9 is substituted with E with K or D at amino acid 545; and in exon 20 H is substituted with R at amino acid 1047.

In some embodiments, methods for determining if a subject is a candidate for treatment with a pan-ErbB tyrosine kinase inhibitor or e.g., neratinib comprise the detection of the mutation in the PIK3CA gene comprises a Polymerase Chain Reaction (PCR) assay, direct sequencing of the PIK3CA gene; sequencing of a cDNA generating from a sample in the patient.

In some embodiments, methods for determining if a subject is a candidate for treatment with a pan-ErbB tyrosine kinase inhibitor or e.g., neratinib comprise the detection of PTEN expression by one or more of: reverse phase protein array, western blotting, semi-quantitative or quantitative IHC.

### DETAILED DESCRIPTION

The disclosure provides assays to determine pathway activation using combined approaches genetic, genomic, and protein biomarkers to accurately characterize "*PI3K activated*" tumors. Such a combined approach to pathway status can be assessed using a statistical stratification of patients in a randomized trial into "*pathway on*" and "*pathway off*' subsets to compare the treatment effect in each arm. Additionally, determining the pathway on versus pathway off status can help select a treatment protocol for a patient suffering from breast cancer. In some embodiments, the treatment protocol selected comprises administering neratinib to a breast cancer patient.

Current strategies for identifying patient usually use a single biomarker to identify the patient populations of interest, i.e. Her2+ or *KRAS* mutant. At the PI3K node, however, the identification of patients' tumors that rely on this signaling node is not simple, because two different protein complexes are involved in this "switch genes involved that have multiple mechanisms of activation (*PIK3CA*) and inactivation (*PTEN*) that result in the same phenotype, i.e. accumulation of PIP₃, which is a second messenger which accumulates in the internal membrane surface forming the binding/docking site for PDK1 and Akt/PKB, then leading to the proliferation and anti-apoptosis signal being conducted to the cell.

Instead of considering individual biomarkers for their predictive ability, this strategy discloses the use of a collection of biomarkers to identify a specific "*pathway* on" patient population that will have clinical benefit from administration of a particular therapeutic pathway inhibitor.

Classification of tumors according to, e.g., mutation analysis, DNA copy number, methylation status, and patterns of gene or protein expression are available. Nearly half of all new oncology compounds approved by the U.S. Food and Drug Administration since the approval of trastuzumab have been associated with some form of patient selection biomarker. These examples primarily focus on measuring target biology in tumor samples. A more recent development in patient selection is the identification of drug resistance mechanisms in an effort to distinguish those patients who will achieve clinical benefit from a specific agent from those who will not (e.g., V-Ki-ras2 Kirsten rat sarcoma [*KRAS*] mutation status identifies those patients who will not benefit from the addition of antibody-based epidermal growth factor receptor (EGFR) inhibitors in colon cancer (1)

Members of the ErbB RTK family (*EGFR, HER2, HER3, HER4*) undergo genetic events leading to signaling activation in multiple human cancer types; those most often noted in breast cancer include amplifications, mutations, and more recently, intronic repeats with a role in transcriptional activation (2-4). PI3K is one of several signaling cascades engaged by the oncogenic RTK complexes at the membrane and may represent a key therapeutic target (recently reviewed in (5). The critical role of this signaling node in cancer is highlighted by the proportion of human malignancies with genetic lesions in genes encoding the components of the cascade, namely *PIK3CA, PTEN, PDK1,* and *AKT.*

Genetic lesions that lead to constitutive pathway activation in various tumors are on opposite fronts. For example, gain-of-function or activating mutations in or amplification of the p110α subunit of the *PIK3CA* gene are observed in some tumors and act as the "accelerators" of the signaling cascade, whereas loss-of-function events (i.e., deletion, promoter methylation, or mutations) are generally seen for *PTEN* and act as the "brakes" on the system.

Current therapeutic approaches in breast cancer that target this pathway include ErbB pathway inhibitors (e.g., trastuzumab, lapatinib, neratinib, BIBW2992), PI3K inhibitors (e.g., XL147, PX-866), mTOR inhibitors (e.g., temsirolimus, everolimus), and dual PI3K-mTOR inhibitors (e.g., BEZ235). The activation of the PI3K pathway has been associated with resistance to ErbB2-targeted therapy in breast cancer, as well as resistance to cytotoxics. Given that multiple therapeutic options exist and that PI3K activity predicts drug resistance in many settings, the question arises as to whether assays can be developed that allow for the prediction of "PI3K pathway activation" in preserved human tumor tissue samples for clinical development. The challenges in developing a single PI3K pathway activation biomarker primarily stem from two key issues. First, a single *key* biomarker has yet to be identified that will specifically measure oncogenic pathway activation. While several such biomarkers have been proposed, each is associated with specific challenges; for example (1) Akt phosphorylation is not an entirely specific marker for this signaling node at PI3K and may not completely capture PI3K activation in all tumor samples (6, 7) and (2) tumor-specific levels of PIP₃, the most proximal pathway marker, may pose a challenge in the setting of preserved tissues, where accurate measurement of phosphorylated lipids may be more difficult than that of phosphorylated proteins (8).

Novel biomarkers aimed at capturing the underlying biology of pathway activation, such as gene expression profiling, represent promising approaches to measuring pathway activation. Clinical strategies are being developed to answer questions related to biopsy timing and the feasibility of genomic approaches in clinical development paradigms and will help to answer some of these key question in the near future. Nonetheless, such approaches currently remain challenging to implement in the setting of global phase 3 trials. In this setting, it will be imperative to develop panels of assays that are applicable in preserved tumor specimens and performed globally in a homogeneous manner and under standardized conditions (i.e., good laboratory practice).

Biomarker discovery for targeted pathway inhibitors in the preclinical setting can employ several distinct approaches, including (1) modeling of drug resistance using panels of xenograft models or cell lines exposed to the drug or (2) modeling of pathway activation after perturbing the pathway in preclinical model systems at the molecular level (e.g., siRNA). Biomarkers derived from such models can be further assessed by measuring pathway markers in human tumor tissues.

Class I_{A} phosphatidylinositol-3-kinase (PI3K) is a heterodimeric lipid kinase complex with two subunits, the p110α catalytic domain and the p85 regulatory domain. Upon ligand binding and receptor tyrosine kinase (RTK) autophosphorylation, PI3K is recruited to the cell membrane, binds to the intracellular arm of the RTK, and catalyzes the conversion of phosphatidylinositol (4,5)-diphosphate (PIP₂) to phosphatidylinositol (3,4,5)-triphosphate (PIP₃).

Under normal physiologic conditions, PI3K plays a key role in the regulation of cellular processes, such as proliferation, migration, and apoptosis. Akt/PKB and phosphoinositide-dependent kinase-1 (PDK1) are recruited to the membrane and activated by direct binding to the accumulated pool of PIP₃. Active PDK1 propagates signaling via phosphorylation of substrates (Akt/PKB, SGK3). Akt/PKB is phosphorylated by both PDK1 (at site T308) and PDK2/mammalian target of rapamycin (mTOR) C2 (at site S473), leading to full activation of Akt/PKB downstream signaling, which leaves Akt/PKB both upstream and downstream of mTOR (6, 9-11).

In an elegant signaling "switch" mechanism at the PI3K-PTEN node, the kinase activity of the PI3K complex is opposed by the dual phosphatase known as phosphatase and tensin homologue deleted on chromosome 10 (*PTEN*), which converts PIP₃ to PIP₂ and essentially functions as a "check" on the activity of PI3K.

Neratinib (also called HKI-272) inhibits phosphorylation of the ErbB receptors and downstream substrates; due to this activity in preclinical models, neratinib has been shown to inhibit phosphorylation and activation of the PI3K complex.. (See, e.g., WO09/052264 at pages 6-7; US2007/0104721 at paragraphs 7 and 21; and U.S. Patent No. 7,399,865).

A decrease in PTEN protein expression and/or in the *PIK3CA* gene have been associated with resistance to treatment of breast cancer with trastuzumab. Using a semiquantitative immunohistochemistry (IHC) assay, these changes have been associated with trastuzumab resistance in breast cancer. Berns K, et al. Cancer Cell. 2007 Oct;12(4):395-402 (12).

### Patterns of PI3K pathway activation in human malignancies

PI3K pathway aberrations are present at diagnosis in a significant percentage of breast cancer patients and data suggest that these represent de novo resistance mechanisms to standard therapy. Importantly, the introduction of a novel targeted therapy (such as a pan-ErbB inhibitor) may restore sensitivity to some standard therapies. The concept behind this type of biomarker strategy is to identify a biologic subset of patients that are predicted to be resistant to the standard of care therapy, where the addition or substitution of the novel pathway inhibitor would be expected to have greater therapeutic efficacy by overcoming that resistance mechanism. For example, in Her2+ breast cancer, PI3K pathway activation predicts resistance to trastuzumab (12-15). Biomarkers of PI3K pathway activation that differentiate two patient subsets (e.g., "PI3K ON" and "PI3K OFF") is used to identify patients predicted to have a response to standard trastuzumab therapy ("PI3K OFF") and those who might require treatment with novel pathway inhibitors (e.g., pan-ErbB inhibitors, in the setting of "PI3K ON") to achieve a clinical response. (49)

Multiple genetic and epigenetic events in tumor cells lead to a common path: accumulation of PIP₃ levels at the cell membrane that leads to enhanced downstream signaling. The goal of a biomarker strategy incorporating PI3K activation is to develop a series of assays that will be able to differentiate patients and group them into distinct subsets based on the presence of tumors that are (1) driven by or dependent on downstream signaling via PI3K or (2) not dependent on this signaling pathway. The combined assessment of *PIK3CA* mutations and *PTEN* loss has demonstrated that PI3K pathway activation is a resistance mechanism to trastuzumab therapy in patients with metastatic ErbB2+ breast cancer (12). To apply such an approach in clinical development and treatment paradigms, a distinct strategy is provided to evaluate the appropriateness of the use of neratinib as a therapy of choice alone or in combination with another agent for the treatment of breast cancer and in one embodiment for treatment of breast cancer.

### PI3K activation

The known genetic events observed in primary breast cancer samples in the *PIK3CA* gene leading to pathway activation are composed of hotspot mutations in exons 9 or 20, gene amplification, or the combination of both.

### PTEN loss

Loss of PTEN has been routinely studied in the clinic using standard IHC approaches, typically with an antibody that recognizes a C-terminal protein epitope caused by mutations that can produce truncated forms of the protein. Various examples of concordance versus discordance between known genetic loss events and the expression of PTEN via IHC exist in the literature; this can lead to some challenges in the interpretation of the underlying biology (16-17). Several potential explanations exist for the discordance between the percentage of patients with genetic lesions and that with decreased protein levels. Without being bound by theory, IHC methods can be qualitative or semiquantitative and differences in interpretation can lead to different results. IHC methods detect all species of the full-length protein (functional or dysfunctional) and "reduced" protein levels may derive from either destabilizing mutations, miRNA expression, or co-expressed stabilizing proteins, whereas a full complement of the PTEN protein can be observed with a point mutation in the phosphatase domain (18-19).

In some embodiments, neratinib is administered to a subject at a dose between 100 and 500 mg per day, between 200 and 400 mg per day, and at a dose of about 250 mg per day.

In some embodiments, the invention provides a method of treating breast cancer with neratinib in conjunction with another treatment for breast cancer. Additional treatment or treatments can include surgery, radiation or additional chemotherapy agents selected from one or more of the following: aromatase inhibitors, including letrozole (Femara), anastrazole (Arimidex), fulvestrant (Faslodex) and exemestane (Aromasin); goserelin (Zoladex); anthracyclines, including doxorubicin (Adriamycin), epirubicin (Ellence), and liposomal doxorubicin (Doxil); taxanes, including docetaxel (Taxotere), paclitaxel (Taxol), and protein-bound paclitaxel (Abraxane), Cyclophosphamide (Cytoxan); Capecitabine (Xeloda) and 5 fluorouracil (5 FU); Vinorelbine (Navelbine); Gemcitabine (Gemzar); and Trastuzumab (Herceptin).

"Inhibition" of PI3K activity can be direct, as in via preventing the complex from binding to substrate and or sequestering of the enzyme, or indirect, as in preventing transcription or translation of the *PIK3CA* gene. In some embodiments, inhibition of PI3K activity comprises administering a pan-ErbB tyrosine kinase inhibitor, e.g., neratinib. As used herein, "intracellular inhibition" of PI3K indicates that the PI3K complex is prevented from activity by direct interference with the PI3K pathway inside the cell, as opposed to an inhibition that occurs via blocking binding or inactivation of a transmembrane cell receptor, e.g., as in inhibition with trastuzumab.

The term "treating," as used herein, unless otherwise indicated, means reversing, alleviating, inhibiting the progress of, or preventing the disorder or condition to which such term applies, or one or more symptoms of such disorder or condition. The term "treatment", as used herein, unless otherwise indicated, refers to the act of treating as "treating" is defined immediately above. As used herein, "subject" and "patient" are used interchangeably.

Quantitative assessment of PTEN protein expression: Standard IHC methods are used to stain tumors for PTEN protein expression. Digital images are obtained and OD scores for both normal tissue (e.g. stromal or endothelial cell) PTEN, as well as tumor PTEN compartments are obtained. The sample's PTEN score is calculated as tumor PTEN OD/normal tissue PTEN OD. A range of tumor PTEN scores are presented with slight differences in normal tissue (e.g. stromal) PTEN expression. Normalization allows for correction in staining differences as an internal control. PTEN, phosphatase and tensin homolog deleted on chromosome 10; OD, optical density.

All references noted herein are incorporated in their entirety.

### EXAMPLES

The present invention will be understood more readily by reference to the following examples, which are provided by way of illustration and are not intended to be limiting of the present invention.

### EXAMPLE 1

### MUTATIONS IN PIK3CA GENE

Activating mutations in the *PIK3CA* gene (which encodes the p110α subunit of the class I_{A} PI3K complex) have been found in a number of human malignancies, including breast, ovarian, lung, esophagus, endometrial, and thyroid cancers.

In breast cancer, mutations in *PIK3CA* have been observed in approximately one quarter of patients in different cohorts tested (range, 8%-40%). Most mutations in breast cancer have been found to cluster in either the kinase or helical domains in exons 9 and 20 of the *PIK3CA* gene. These gain-of-function mutations disrupt folding interactions in the p110α unit and the interface between the p110α and p85 subunits, leading to structural changes in the kinase domain that result in increased enzymatic activity.

Other mutations that have been detected in global screens of *PIK3CA* exons are observed with less frequency in the breast cancer population and have not been shown to have the same PI3K activation biology. More than 80% of the mutations identified in breast cancer can be detected by assaying for certain hotspot mutations in exon 9 (E542K, E545K, E545D) and in exon 20 (H1047R) (20).

Both helical and kinase domain mutations in exons 9 and 20 lead to a gain of PI3K signaling activity. Studies in breast cancer patients have shown that *PIK3CA* mutations in total, or specific groups with exon 9 or 20 mutations, have a negative prognostic value. Helical and kinase domain mutations may have different predictive value as well; exon 9 mutations alone predict enhanced sensitivity to the combination of everolimus and letrozole (vs. letrozole alone) in the neoadjuvant setting.

Activating mutations in exons 9 and 20 (E542K, E545D, E545K, and H1047R) are measured by allele-specific polymerase chain reaction (PCR).

### EXAMPLE 2

### AMPLIFICATION OF THE PIK3CA GENE

The *PIK3CA* gene (3q26.3 locus) has also been shown to undergo amplification in a number of tumors and, similar to gain-of-function mutations, amplification correlates with poor prognosis (21-24). *PIK3CA* amplification is one of the key mechanisms of PI3K pathway activation in ovarian and endometrial cancers; in these patients, amplification leads to increased gene dosage and increased pathway activity and correlates with resistance to standard therapy and poor prognosis (21, 22, 25, 26). *PIK3CA* amplifications are observed with less frequency in breast cancer. In initial diagnostic samples, 8.7% of patients were found to have a chromosomal gain at 3q26 *(PIK3CA* at this locus); half of those patients also harbored *PIK3CA* mutations (27). High-level amplifications were observed in a group of breast cancer samples identified as basal subtype by expression profiling (28). Breast cancer cell lines were found to harbor *PIK3CA* amplifications; co-existence of both amplification and mutation of the *PIK3CA* gene results in increased pathway activation measured by enhanced phosphorylation of Akt.

Gene amplification can be determined using fluorescence in situ hybridization (FISH) (20)

### EXAMPLE 3

### PTEN EXPRESSION

The tumor suppressor PTEN is a dual-specificity phosphatase (lipid and protein) that functions as a check (or the "brakes") on the PI3K signaling complex. PTEN mediates the dephosphorylation of PIP₃ to PIP₂, eliminating the membrane binding site for PDK1 and Akt/PKB and thus antagonizing the activity of PI3K. The *PTEN* gene (at locus 10q23) is inactivated in a number of human malignancies, including breast, brain, endometrial, kidney, and prostate cancers (29-32) The inactivation of *PTEN* correlates with disease progression and poor prognosis, suggesting a key role in oncogenesis (16, 33-34). In experimental systems, the inactivation of *PTEN* has been shown to lead to unchecked activation of Akt/PKB and subsequently to an oncogenic phenotype by inhibition of apoptosis whereas restoration of *PTEN* expression in *PTEN*-null systems leads to loss of the oncogenic phenotype (32, 35). Unchecked Akt/PKB activity leads to inhibition of apoptosis, cellular growth, and enhanced proliferation [36].

In breast cancer, multiple mechanisms of *PTEN* loss of function have been demonstrated, including mutations, gene deletions, and transcriptional downregulation via miRNA or epigenetic silencing. Reduction in PTEN protein levels in breast cancer is observed using immunohistochemistry (IHC); various studies have reported reduced PTEN in 15% to 48% of patients (34, 37-40). The spectrum of *PTEN* mutations, gene deletions, and epigenetic events as mechanisms of inactivation present an interesting study of tumor biology, and the variable combinations of these inactivation mechanisms are likely to contribute to the heterogeneity in published literature on the reduction in PTEN expression observed. Mutations in the *PTEN* gene are quite common in malignancies, such as endometrial carcinoma and glioblastoma; however, such mutations are relatively rare in breast cancer (found in only approximately 5% of patients and most represent frame shift mutations that can lead to a destabilized protein) 30, 41-42). In contrast, the major mechanism of *PTEN* inactivation in breast cancer appears to be *PTEN* gene deletion (37). Multiple additional mechanisms of *PTEN* loss beyond gene loss or mutations have been identified. At the transcriptional level, epigenetic silencing via promoter methylation or miRNA expression (e.g., miR-21) has been described (43-45). Further mechanisms to reduce PTEN expression involve loss of stabilizing proteins, such as Rak, which phosphorylates PTEN, thus protecting it from ubiquitin-mediated degredation (19). As used herein, "positive for the presence of a decrease in PTEN protein expression" means a decrease in PTEN expression levels as compared to non tumorigenic tissue (e.g., non-tumorigenic stromal or endothelial tissue).

Alternative methods to evaluate PTEN protein expression are contemplated for use in the practice of the invention. Quantitative methods, such as reverse-phase protein microarray technology or a quantitative IHC method, can allow detection of minor changes in protein levels that are not detected by standard IHC. These methods have shown a better concordance between interpretation of PTEN protein levels and genetics (19, 46, 47). These novel quantitative protein measurements are applicable in preserved samples and such assays are potentially more reliable in studying the underlying pathway biology compared with standard immunohistocytochemistry.

### EXAMPLE 4

### SELECTION OF PATIENT FOR NERATINIB THERAPY

A sample is obtained from a patient with breast cancer. The sample is analyzed for the presence or absence of one or more of PIK3CA gene amplification; a mutation in PIK3CA; and a decrease in PTEN protein expression. The presence of one or more of these (PIK3CA gene amplification; a mutation in PIK3CA; and a decrease in PTEN protein expression) results in the patient being designated as having a tumor that is "PI3K ON." If a patient is designated as "PI3K ON", then the patient is treated with neratinib. As used herein, any clinical benefit associated with the neratinib or therapeutic combination can be compared with that seen in the standard of care treatment group. This can be done by making comparisons either in each group of patients separately or for a given treatment between each group of patients using linear regression models. These comparisons can identify the population for whom the neratinib represents substantial improvement over standard of care (presumably because of some level of tumor "dependence" on the pathway).

### EXAMPLE 5

### PI3K PATHWAY ACTIVATION AS A PREDICTIVE BIOMARKER FOR PATIENT SELECTION: STATISTICAL CONSIDERATIONS IN THE CLINIC

The hypothesis for incorporating the biomarker strategy of the present invention in a clinical trial is that patients expected to have a clinically meaningful response to a particular drug-or combination of drugs-superior to that of a comparator agent or the standard of care will be prospectively selected. In randomized clinical trials, this approach would enrich the patient population for responders in the experimental arm because the selection is based on the underlying biology of the therapeutic agent. In contrast, enriching the patient population purely for favorable responses will not impact the outcome of the randomized trial, as both experimental and control arms will have more favorable outcomes. Additionally, such patient selection approaches using the underlying biology of the tumor in future trials might also provide rational alternative therapeutic options for those patients whose tumors are predicted to be resistant to a particular drug or therapeutic combination and would be excluded from a given trial. For example, with the knowledge that PI3K activation is a marker of resistance to trastuzumab (12, 14, 48), it would be optimal to have alternative treatments available, such as the tyrosine kinase inhibitor class of agents (e.g., the irreversible pan-ErbB inhibitor, neratinib, or the reversible Her1/Her2 inhibitor, lapatinib). (49)

Two groups of patients are created within a randomized trial-one group of patients in which PI3K pathway activation is apparent in the tumor sample (i.e., "PI3K ON" or patients with the presence of one or more of these: PIK3CA gene amplification; a mutation in PIK3CA; and a decrease in PTEN protein expression) and another group with no evidence of PI3K activation (i.e., "PI3K OFF" or patients with the absence of all three of these: PIK3CA gene amplification; a mutation in PIK3CA; and a decrease in PTEN protein expression). Active PI3K ("PI3K ON") can be defined as *"PIK3CA* mutation +" and/or *"PIK3CA* gene amplification" and/or "PTEN loss" and/or "PTEN low." Based on preliminary biomarker data obtained prior to the clinical trial (to support its predictability of response), such biomarkers can be considered as exploratory endpoints or as secondary endpoints with stratification. Such a grouping of the patients in a randomized trial could be treated as a separate level of stratification in the trial, with a different null hypothesis than standard geographic or prior treatment group stratifications (where the null hypothesis is that differences exist in the strata). For such a pathway grouping stratification, the null hypothesis would be that no difference exists in the treated group.

At study enrollment, patient selection biomarkers are measured in each patient; in this example, tumors are assessed by phosphatase and tensin homolog deleted on chromosome 10 (PTEN) immunohistochemistry, *PIK3CA* mutations, and *PIK3CA* fluorescence in situ hybridization (FISH). The group of patients defined here as "PI3K ON" is *"PIK3CA* mutant" or *"PIK3CA* amplified" or "PTEN null" or "PTEN reduced." In this example, "PI3K OFF" is defined as *"PIK3CA* wild-type and non-amplified," and "PTEN normal." PI3K ON patients are treated with neratinib. The clinical benefit can then be compared between these two populations using linear regression methods. The null hypothesis is that the differential treatment effect in the "PI3K ON" group is the same as the differential treatment effect in the "PI3K OFF" group.

In this type of patient selection approach, the null hypothesis is that the differential treatment effect in the "PI3K ON" group is the same as the differential treatment effect in the "PI3K OFF" group. The clinical benefit can be compared between these two populations using linear regression methods. This approach might indicate that a drug is most useful for patients with defined activation events of a given pathway (such as presented here for PI3K). Although such an approach carries a perceived risk of further subdividing the existing subsets (e.g., "PI3K ON" and "PI3K OFF" subsets in Her2+ breast cancer), it may allow the identification of those patients who remain at risk of relapse despite the standard regimen and accurately define the adjuvant treatment regimens based on underlying biology at the initial diagnosis (when the patients remain curable). Given the differences in the underlying tumor biology associated with various biomarkers, as well as key reports of downstream signaling differences, alternate subsets of biomarkers may identify responder populations more accurately than the global definition of "PI3K ON" proposed previously. For example, pan-ErbB inhibitors may be exquisitely effective in patients with tumors defined as "PTEN loss" or "PTEN low," whereas PI3K inhibitors may have less activity against "PTEN loss" tumors and increased efficacy in tumors harboring *PIK3CA* mutations or amplifications.

### REFERENCES

1. Allegra CJ, Jessup JM, Somerfield MR et al (2009) American Society of Clinical Oncology provisional clinical opinion: testing for KRAS gene mutations in patients with metastatic colorectal carcinoma to predict response to anti-epidermal growth factor receptor monoclonal antibody therapy. J Clin Oncol 27:2091-2096.
2. Hynes NE, Lane HA (2005) ERBB receptors and cancer: the complexity of targeted inhibitors. Nat Rev Cancer 5:341-354.
3. Zhou Q, Cheung YB, Jada SR et al (2006) EGFR Intron 1 polymorphism in Asian Populations and its correlation with EGFR gene expression and amplification in breast tumor tissues. Cancer Biol Ther 5:1445-1449.
4. Buerger H, Packeisen J, Boecker A et al (2004) Allelic length of a CA dinucleotide repeat in the egfr gene correlates with the frequency of amplifications of this sequence--first results of an inter-ethnic breast cancer study. J Pathol 203:545-550.
5. Engelman JA (2009) Targeting PI3K signalling in cancer: opportunities, challenges and limitations. Nat Rev Cancer 9:550-562.
6. Vasudevan KM, Barbie DA, Davies MA et al (2009) AKT-independent signaling downstream of oncogenic PIK3CA mutations in human cancer. Cancer Cell 16:21-32.
7. Blanco-Aparicio C, Renner O, Leal JF et al (2007) PTEN, more than the AKT pathway. Carcinogenesis 28:1379-1386.
8. Maehama T, Taylor GS, Slama JT et al (2000) A sensitive assay for phosphoinositide phosphatases. Anal Biochem 279:248-250.
9. Guertin DA, Stevens DM, Thoreen CC et al (2006) Ablation in mice of the mTORC components raptor, rictor, or mLST8 reveals that mTORC2 is required for signaling to Akt-FOXO and PKCalpha, but not S6K1. Dev Cell 11:859-871.
10. Stokoe D, Stephens LR, Copeland T et al (1997) Dual role of phosphatidylinositol-3,4,5-trisphosphate in the activation of protein kinase B. Science 277:567-570.
11. Yuan TL, Cantley LC (2008) PI3K pathway alterations in cancer: variations on a theme. Oncogene 27:5497-5510.
12. Berns K, Horlings HM, Hennessy BT et al (2007) A functional genetic approach identifies the PI3K pathway as a major determinant of trastuzumab resistance in breast cancer. Cancer Cell 12:395-402.
13. Eichhorn PJ, Gili M, Scaltriti M et al (2008) Phosphatidylinositol 3-kinase hyperactivation results in lapatinib resistance that is reversed by the mTOR/phosphatidylinositol 3-kinase inhibitor NVP-BEZ235. Cancer Res 68:9221-9230.
14. Nagata Y, Lan KH, Zhou X et al (2004) PTEN activation contributes to tumor inhibition by trastuzumab, and loss of PTEN predicts trastuzumab resistance in patients.[see comment]. Cancer Cell 6:117-127.
15. Bedard PL, Cardoso F, Piccart-Gebhart MJ (2009) Stemming resistance to HER-2 targeted therapy. J Mammary Gland Biol Neoplasia 14:55-66.
16. Bose S, Crane A, Hibshoosh H et al (2002) Reduced expression of PTEN correlates with breast cancer progression. Hum Pathol 33:405-409.
17. Bettendorf O, Schmidt H, Staebler A et al (2008) Chromosomal imbalances, loss of heterozygosity, and immunohistochemical expression of TP53, RB1, and PTEN in intraductal cancer, intraepithelial neoplasia, and invasive adenocarcinoma of the prostate. Genes Chromosomes Cancer 47:565-572.
18. Maehama T (2007) PTEN: its deregulation and tumorigenesis. Biol Pharm Bull 30:1624-1627.
19. Yim EK, Peng G, Dai H et al (2009) Rak functions as a tumor suppressor by regulating PTEN protein stability and function. Cancer Cell 15:304-314.
20. Board RE, Thelwell NJ, Ravetto PF et al (2008) Multiplexed assays for detection of mutations in PIK3CA. Clin Chem 54:757-760.
21. Salvesen HB, Carter SL, Mannelqvist M et al (2009) Integrated genomic profiling of endometrial carcinoma associates aggressive tumors with indicators of PI3 kinase activation. Proc Natl Acad Sci USA 106:4834-4839.
22. Woenckhaus J, Steger K, Sturm K et al (2007) Prognostic value of PIK3CA and phosphorylated AKT expression in ovarian cancer. Virchows Arch 450:387-395.
23. Wu G, Mambo E, Guo Z et al (2005) Uncommon mutation, but common amplifications, of the PIK3CA gene in thyroid tumors. J Clin Endocrinol Metab 90:4688-4693.
24. Redon R, Muller D, Caulee K et al (2001) A simple specific pattern of chromosomal aberrations at early stages of head and neck squamous cell carcinomas: PIK3CA but not p63 gene as a likely target of 3q26-qter gains. Cancer Res 61:4122-4129.
25. Shayesteh L, Lu Y, Kuo WL et al (1999) PIK3CA is implicated as an oncogene in ovarian cancer. Nat Genet 21:99-102.
26. Ma YY, Wei SJ, Lin YC et al (2000) PIK3CA as an oncogene in cervical cancer. Oncogene 19:2739-2744.
27. Wu G, Xing M, Mambo E et al (2005) Somatic mutation and gain of copy number of PIK3CA in human breast cancer. Breast Cancer Res 7:R609-616.
28. Adelaide J, Finetti P, Bekhouche I et al (2007) Integrated profiling of basal and luminal breast cancers. Cancer Res 67:11565-11575.
29. Steck PA, Pershouse MA, Jasser SA et al (1997) Identification of a candidate tumour suppressor gene, MMAC1, at chromosome 10q23.3 that is mutated in multiple advanced cancers. Nature Genetics 15:356-362.
30. Li J, Yen C, Liaw D et al (1997) PTEN, a putative protein tyrosine phosphatase gene mutated in human brain, breast, and prostate cancer.[see comment]. Science 275:1943-1947.
31. Li DM, Sun H (1997) TEP1, encoded by a candidate tumor suppressor locus, is a novel protein tyrosine phosphatase regulated by transforming growth factor beta. Cancer Res 57:2124-2129.
32. Li DM, Sun H (1998) PTEN/MMAC1/TEP1 suppresses the tumorigenicity and induces G1 cell cycle arrest in human glioblastoma cells. Proc Natl Acad Sci USA 95:15406-15411.
33. Rubin MA, Gerstein A, Reid K et al (2000) 10q23.3 loss of heterozygosity is higher in lymph node-positive (pT2-3,N+) versus lymph node-negative (pT2-3,N0) prostate cancer. Hum Pathol 31:504-508.
34. Depowski PL, Rosenthal SI, Ross JS (2001) Loss of expression of the PTEN gene protein product is associated with poor outcome in breast cancer. Mod Pathol 14:672-676.
35. Lu Y, Lin YZ, LaPushin R et al (1999) The PTEN/MMAC1/TEP tumor suppressor gene decreases cell growth and induces apoptosis and anoikis in breast cancer cells. Oncogene 18:7034-7045.
36. Samuels Y, Ericson K (2006) Oncogenic PI3K and its role in cancer. Curr Opin Oncol 18:77-82.
37. Bose S, Wang SI, Terry MB et al (1998) Allelic loss of chromosome 10q23 is associated with tumor progression in breast carcinomas. Oncogene 17:123-127.
38. Saal LH, Holm K, Maurer M et al (2005) PIK3CA mutations correlate with hormone receptors, node metastasis, and ERBB2, and are mutually exclusive with PTEN loss in human breast carcinoma. Cancer Res 65:2554-2559.
39. Perez-Tenorio G, Alkhori L, Olsson B et al (2007) PIK3CA mutations and PTEN loss correlate with similar prognostic factors and are not mutually exclusive in breast cancer. Clin Cancer Res 13:3577-3584.
40. Perren A, Weng LP, Boag AH et al (1999) Immunohistochemical evidence of loss of PTEN expression in primary ductal adenocarcinomas of the breast. Am J Pathol 155:1253-1260.
41. Maehama T, Taylor GS, Dixon JE (2001) PTEN and myotubularin: novel phosphoinositide phosphatases. Annu Rev Biochem 70:247-279.
42. Ali IU, Schriml LM, Dean M (1999) Mutational spectra of PTEN/MMAC1 gene: a tumor suppressor with lipid phosphatase activity. J Natl Cancer Inst 91:1922-1932.
43. Garcia JM, Silva J, Pena C et al (2004) Promoter methylation of the PTEN gene is a common molecular change in breast cancer. Genes Chromosomes Cancer 41:117-124.
44. Meng F, Henson R, Wehbe-Janek H et al (2007) MicroRNA-21 regulates expression of the PTEN tumor suppressor gene in human hepatocellular cancer. Gastroenterology 133:647-658.
45. Huang TH, Wu F, Loeb GB et al (2009) Upregulation of miR-21 by HER2/neu signaling promotes cell invasion. J Biol Chem.
46. Stemke-Hale K, Gonzalez-Angulo AM, Lluch A et al (2008) An integrative genomic and proteomic analysis of PIK3CA, PTEN, and AKT mutations in breast cancer. Cancer Res 68:6084-6091.
47. Zhou J, Wulfkuhle J, Zhang H et al (2007) Activation of the PTEN/mTOR/STAT3 pathway in breast cancer stem-like cells is required for viability and maintenance. Proc Natl Acad Sci USA 104:16158-16163.
48. Yang H, Kong W, He L et al (2008) MicroRNA expression profiling in human ovarian cancer: miR-214 induces cell survival and cisplatin resistance by targeting PTEN. Cancer Research 68:425-433.
49. O'Brien N et al (2010) Activated Phosphoinositide 3-Kinase/AKT Signaling Confers Resistance to Trastuzumab but not Lapatinib. Mol Cancer Ther 9(6): 1489-1502

**The application discloses *inter alia* the following items:**
1. A method for treating breast cancer in a subject which comprises
   a) obtaining a sample from the subject;
   b) detecting the presence or absence of one or more of PIK3CA gene amplification; a mutation in PIK3CA; and a decrease in PTEN protein expression; and
   treating a subject that is positive for the presence of one or more of PIK3CA gene amplification; a mutation in PIK3CA; and a decrease in PTEN protein expression by administering to the subject a pan-ErbB tyrosine kinase inhibitor.
2. The method of item 1 wherein the pan-ErbB tyrosine kinase inhibitor is irreversible and prevents binding of PIK3CA to the intracellular portion of ErbB
3. The method of item 2 wherein the irreversible pan-ErbB tyrosine kinase inhibitor is neratinib.
4. The method of any of items 1-3 wherein the mutation in the PIK3CA gene comprises one or more of the following point mutations: in exon 9 E is substituted with K at position 542 of the mature protein sequence; E with K or D at amino acid 545; and in exon 20 H is substituted with R at amino acid 1047 of the mature protein sequence.
5. The method of any of items 1-4 wherein the method of the detection of the mutation in the PIK3CA gene comprises a Polymerase Chain Reaction (PCR) assay, or direct nucleic acid sequencing or hybridization with a nucleic acid probe specific for the PIK3CA gene.
6. The method of any of items 1-5 wherein the detection of PTEN expression comprises one or more of: reverse phase protein array, western blotting , semi-quantitative or quantitative immunohistochemistry (IHC).
7. The method of any of items 1-6 which further comprises administering one or more compositions or therapies to the subject if the subject is positive for the presence of one or more of PIK3CA gene amplification; a mutation in PIK3CA; and a decrease in PTEN protein expression: surgery, radiation or additional chemotherapy agents selected from one or more of the following: aromatase inhibitors, including letrozole (Femara), anastrazole (Arimidex), fulvestrant (Faslodex) and exemestane (Aromasin); goserelin (Zoladex); anthracyclines, including doxorubicin (Adriamycin), epirubicin (Ellence), and liposomal doxorubicin (Doxil); taxanes, including docetaxel (Taxotere), paclitaxel (Taxol), and protein-bound paclitaxel (Abraxane), Cyclophosphamide (Cytoxan); Capecitabine (Xeloda) and 5 fluorouracil (5 FU); Vinorelbine (Navelbine); Gemcitabine (Gemzar);Trastuzumab (Herceptin), lapatinib, BIBW2992, PI3K inhibitors (e.g., XL147, PX-866), mTOR inhibitors (e.g., temsirolimus, everolimus), and dual PI3K-mTOR inhibitors (e.g., BEZ235).
8. The method of item 1 wherein if the sample from the subject is negative for PIK3CA gene amplification; a mutation in PIK3CA; and a decrease in PTEN protein expression the subject is administered Trastuzumab.
9. A method of determining if a subject with breast cancer is a candidate for treatment with neratinib which comprises:
   a) obtaining a sample from the subject;
   b) detecting the presence or absence of PIK3CA gene amplification;
   wherein if the subject is positive for the presence of one or more of the following: PIK3CA gene amplification; a mutation in PIK3CA; and a decrease in PTEN protein expression;
   the subject is identified as a candidate for treatment with a pan-Erb tyrosine kinase inhibitor.
10. The method of item 9 wherein the pan-ErbB tyrosine kinase inhibitor is irreversible and prevents binding of PIK3CA to the intracellular portion of ErbB
11. The method of item 10 wherein the irreversible pan-ErbB tyrosine kinase inhibitor is neratinib.
12.The method of any of items 9-11 wherein the mutation in the PIK3CA gene is selected from the following point mutations: in exon 9 E is substituted with K at position 542 of the protein sequence; in exon 9 is substituted with E with K or D at amino acid 545; and in exon 20 H is substituted with R at amino acid 1047.
13. The method of any of items 9-12 wherein the detection of the mutation in the PIK3CA gene comprises a Polymerase Chain Reaction (PCR) assay, direct sequencing of the PIK3CA gene; sequencing of a cDNA generating from a sample in the patient.
14. The method of any of items 9-13 wherein the detection of PTEN expression comprises one or more of: reverse phase protein array, western blotting, semi-quantitative or quantitative IHC.

## Claims

1. A composition comprising neratinib for use in a method for treatment of breast cancer in a subject, wherein the subject is positive for the presence of one or more of: PIK3ACA gene amplification, a mutation in PIK3CA and a decrease in PTEN protein expression; and wherein the method further comprises administration of capecitabine.

2. The composition for use of claim 1, wherein the mutation in the PIK3CA gene comprises one or more of the following point mutations: in exon 9, E is substituted with K at position 542 of the mature protein sequence; and E is substituted with K or D at amino acid 545; and in exon 20, H is substituted with R at amino acid 1047 of the mature protein sequence.

3. The composition for use of claim 1 or 2, wherein the mutation in the PIK3CA gene is detected using a Polymerase Chain Reaction assay, or direct nucleic acid sequencing, or hybridization with a nucleic acid probe specific for the PIK3CA gene.

4. The composition for use of any one of claims 1-3, wherein the PTEN expression is detected using one or more of: reverse phase protein array, western blotting, semiquantitative, or quantitative immunohistochemistry.

5. The composition for use of any one of claims 1-4, wherein the method further comprises surgery, radiation or administration of additional chemotherapy agents selected from one or more of the following: aromatase inhibitors; goserelin (Zoladex); anthracyclines; taxanes; cyclophosphamide (Cytoxan); 5 fluorouracil (5 FU); vinorelbine (Navelbine); gemcitabine (Gemzar); trastuzumab (Herceptin); lapatinib; BIBW2992, mTOR inhibitors and dual PI3K-mTOR inhibitors.

6. The composition for use of claim 5, wherein the aromatase inhibitor is letrozole (Femara), anastrazole (Arimidex), fulvestrant (Faslodex), or exemestane (Aromasin).

7. The composition for use of claim 5, wherein the anthracycline is doxorubicin (Adriamycin), epirubicin (Ellence), or liposomal doxorubicin (Doxil).

8. The composition for use of claim 5, wherein the taxane is docetaxel (Taxotere), paclitaxel (Taxol), or protein-bound paclitaxel (Abraxane).

9. The composition for use of claim 5, wherein the mTOR inhibitor is temsirolimus or everolimus.

10. The composition for use of claim 5, wherein the dual PI3K-mTOR inhibitor is BEZ235.

11. The composition for use of any one of claims 1-10, wherein the method comprises administering neratinib at a dose of between 100 and 500 mg per day.

12. The composition for use of any one of claims 1-10, wherein the method comprises administering neratinib at a dose of 250 mg per day.

13. The composition for use of any one of claims 1-12, wherein the breast cancer is resistant to treatment with trastuzumab.
